# DEMANDE DE BREVET EUROPEEN

(11) **EP 2 409 693 A2**
(43) Date de publication de la demande: **25.01.2012**
(21) Numéro de dépôt: 11174443.9
(22) Date de dépôt: 19.07.2011
(51) Int. Cl.: A61K 31/131, A61K 31/145, A61K 31/17, A61K 31/26, A61K 31/27, A61K 31/4453, A61K 31/4468, A61K 31/5375, A61P 17/00

(54) **Isothiocyanates et dérivés pour l'utilisation dans le traitement et/ou la prévention des lucites**

(30) Priorité: 23.07.2010 BE 201000459
(71) Demandeur: Auriga International, 1410 Waterloo (BE)
(72) Inventeur: Dubois, Jacques, 1495 Villers-la-ville (BE); Marchal, Alfred, 1410 Waterloo (BE); Lacroix, Damien, 5030 Ernage (BE); Cabou, Jérôme, 59163 Saint Aybert (FR)
(74) Mandataire: Coulon, Ludivine

(57) **Abrégé**

Utilisation d'un composé de formule générale (I) pour la prévention et/ou le traitement de la lucite

R₇-R₁-R₄-R₂ (I)

dans laquelle R₄ est un groupement sulfinyle, sulfonyle, carbonyle, R₇ représente un groupement amino, isothiocyanato ou carbamothioate de formule -NH(C=S)-R₈ dans laquelle R₈ représente un groupe , amine primaire ou secondaire, alcoolate, ou thiolate et R₁ et R₂ représentent tous deux, indépendamment l'un de l'autre un groupe alkyle, aryle ou alkylaryle éventuellement substitué par un ou plusieurs hétéroatomes.

## Description

La présente invention se rapporte au traitement et/ou à la prévention des lucites.

La lucite désigne des photodermatoses dont le mécanisme d'induction photosensibilisant n'est pas encore identifié dans l'état actuel de nos connaissances. Elles sont typiquement caractérisées par des plaques, vésicules ou papules érythémateuses se développant sur la peau 30 minutes à quelques heures après l'exposition aux UV. En l'absence d'exposition supplémentaire, les éruptions se résorbent pour disparaitre après deux semaines au maximum, laissant dès lors une peau saine.

Les lucites (du latin lux: lumière) sont des éruptions déclenchées par les rayons solaires. La lucite estivale bénigne est la plus fréquente, elle est souvent qualifiée "d'allergie au soleil". La lucite estivale touche le plus souvent la femme jeune et apparaît dès les premiers rayons de soleil. La lucite estivale se traduit par de petits boutons qui démangent au niveau du décolleté et des bras. L'éruption récidive après chaque exposition, mais diminue au fur et à mesure des expositions.

Il est intéressant de noter que les femmes sont atteintes deux à trois fois plus que les hommes. Cette prépondérance pourrait laisser penser à l'implication d'un mécanisme de type hormonal. Cependant, ceci n'a pu être démontré jusqu'à présent. Le pourcentage de la population développant un type de lucite lors de l'exposition aux UV est estimé à 10% au Etats-Unis, 21% en Suède, 15% en Angleterre, et 5% en Australe

La lucite peut affecter tout un chacun, mais il semble que les individus qui ont une peau claire sont les plus sujets à ce type de photodermatose. Un débat est en cours concernant la longueur d'onde du rayonnement induisant les lucites, il existe des preuves incriminant les UVA et les UVB. Néanmoins, il est possible que certains rayons de l'ordre du visible le soient également.

Outre la lucite estivale, il existe d'autres types de lucites :
- La lucite estivale bénigne (LEB)
- La lucite hivernale
- La lucite polymorphe
- La photodermatose printanière juvénile (ou éruption printanière des oreilles)
- L'urticaire solaire
- Autres lucite: Le prurigo actinique, la dermatite actinique chronique, et l'hydroa vacciniforme sont des affections exceptionnelles.

En terme de prévention, il est généralement recommandé d'utiliser les moyens classiques de protection solaire : éviter de s'exposer volontairement au soleil, se protéger avec des vêtements adaptés, utiliser des crèmes solaires, s'exposer progressivement, ...

Les écrans solaires sur le marché filtrent une bonne partie des rayonnements UVB et UVA, mais ils sont souvent mal utilisés (quantité insuffisante, non renouvellement, élimination à l'eau,...). Un écran de très forte protection ou SPF 50+ est généralement recommandé. Cependant, l'utilisation de protection solaire s'avère parfois insuffisante.

D'autres traitements et/ou moyens de prévention existent, mais se révèlent tous d'une efficacité limitée.

Par exemple, il existe un traitement à base de caroténoïdes Les caroténoïdes sont présents dans nombre de compléments alimentaires, ils y sont associés à d'autres agents antioxydants comme vitamine E, vitamine C, sélénium, les huiles de poisson etc. (Photoderm ora®, Oenobiol solaire®, Oxelio®, etc.). Malheuresement, aucune étude ne permet à ces produits de revendiquer une efficacité à ce jour pour prévenir la lucite, mais ils pourraient entraîner un bénéfice chez certains patients. Cependant, leur "dilution" systémique peut faire douter d'une action cutanée ciblée.

Un autre exemple réside dans l'acide para amino-Benzoïque (PABA). Le PABA est parfois utilisé avec de bons résultats pour la prévention de la lucite estivale bénigne. Le dermatologue prescrit par exemple le Pabasun® à la dose de 4 à 6 cp/j (1 comprimé à 500 mg/10 kg de poids), en trois prises pendant les repas. Le traitement sera débuté 15 jours avant le début de la période d'exposition solaire et poursuivi durant celle-ci.

On connait également des traitements à base de vitamine PP (nicotinamide - 83) La vitamine PP est prescrite (Nicobion 500^{®}) avec des résultats variables. Si certains auteurs ont retrouvé une efficacité intéressante, d'autres pensent que ce traitement n'est pas efficace.

Il existe aussi des traitements à base d'antipaludéens de synthèse (Plaquenil^{®}, Nivaquine^{®}) Les antipaludéens de synthèse sont efficaces pour la « prévention des lucites ». Le traitement doit être débuté 7 jours avant le début de l'exposition et poursuivi pendant la durée de la photosensibilité. La prise d'antipaludéens de synthèse nécessite un bilan ophtalmologique avant sa mise en route, mais aussi régulièrement, chaque année, s'il est utilisé plus de trois mois par an.

En outre, certains recommandent les antihistaminiques (Aerius, Xyzall, Kestin...) Ces antihistaminiques sont utiles pour la prévention et/ou le traitement des symptômes de l'urticaire solaire ou des lucites bénignes, un ou deux antihistaminiques peuvent être recommandés.

Le mécanisme exact de cette action n'est pas élucidé. Mais de nombreux essais cliniques ont montré une légère influence positive des antihistaminiques sur la lucite.

Enfin, les ultraviolets dans une photothérapie représentent le traitement préventif qui donne les meilleurs résultats, son efficacité est excellente dans la plupart des cas. Des cures de photothérapie en cabine peuvent être recommandées par le dermatologue pour "préparer la peau au soleil". Les séances de photothérapie permettent de délivrer des doses croissantes de rayons ultraviolets, qui entraînent une tolérance progressive de la peau au soleil. Cependant, il est important de prendre en considération les risques liés à l'utilisation des bancs solaires.

On connait également des traitements curatifs, généralement sous la forme de crème à la cortisone ou leurs analogues appelés les dermocorticoides ou à base d'antihistaminiques. Ces crèmes permettent de soulager les démangeaisons et de diminuer l'intensité de l'éruption. Les crèmes à base d'antihistaminiques sont plutôt efficaces pour diminuer l'intensité des démangeaisons liées aux lucites bénignes et à l'urticaire solaire.

Comme on peut le constater, La lucite est une photodermatose survenant à des degrés divers après une exposition aux rayonnements. Celle-ci disparait après quelques temps. Il n'y a actuellement que des traitements préventifs et palliatifs dont l'efficacité est toute relative.

Il existe donc un besoin d'améliorer l'efficacité des traitements existants curatifs ou de prévention et d'améliorer ainsi le confort des patients qui en sont atteints.

L'invention à pour objet de résoudre ce problème en procurant un traitement curatif ou préventif efficace contre ce type d'affection.

A cette fin, la présente invention propose l'utilisation d'un composé de formule générale (I) pour la prévention et/ou le traitement de la lucite

R₇-R₁-R₄-R₂ (I)

dans laquelle R₄ est un groupement sulfinyle, sulfonyle, carbonyle, R₇ représente un groupement amino, isothiocyanato ou carbamothioate de formule -NH(C=S)-R₈ dans laquelle R₈ représente un groupe , amine primaire ou secondaire, alcoolate, ou thiolate et R₁ et R₂ représentent tous deux, indépendamment l'un de l'autre un groupe alkyle, aryle ou alkylaryle éventuellement substitué par un ou plusieurs hétéroatomes.

Parmi les composés de formule générale (I), on trouve entre autres, le sulforapllane, le sulforamate, l'oxomate, leurs analogues et dérivés, dont le mode d'action auprès des spécialistes les a conduits à la conclusion que sulforaphane, leurs dérivés et analogues pourrait avoir un effet pour la prévention des manifestations de type "lucite".

Le sulforaphane est une molécule naturellement présente dans les crucifères, tel le brocoli ou le chou de Bruxelles. Cette molécule a suscité un grand intérêt dans les années 1990 car on lui attribue l'origine des effets bénéfiques sur la santé de la consommation de ces végétaux, notamment comme anticancéreux. Cette molécule présente une fonction isothiocyanate à laquelle de nombreux effets bénéfiques ont été attribués.

En effet, à ce jour, de nombreuses études ont été réalisées avec le sulforaphane dans le domaine de la protection des cellules contre les agressions et les cancers, cependant aucune d'entre elles ne traite du problème de la lucite.

En général, chez l'homme, les isothiocyanates sont rapidement absorbés et se conjuguent ensuite avec les thiols de protéines, de la cystéine ou du glutathion. Dans l'organisme, cette réaction de conjugaison est un processus réversible et l'adduit peut se dissocier pour libérer à nouveau l'isothiocyanate.

Lorsque les isothiocyanates se conjuguent avec le glutathion, ils sont ensuite métabolisés par la voie des acides mercapturiques pour être finalement excrétés dans les urines principalement sous la forme d'adduits de la N-acétyl cystéine.

Comme décrit ci-dessus, c'est donc la fonction isothiocyanate qui est impliquée dans la métabolisation du sulforaphane. Elle réagit notamment avec le glutathion et les thiols des acides aminés.

C'est notamment grâce à ce type d'interactions que le sulforaphane peut induire les nombreux effets qui lui sont attribués (par exemple : stimulation des enzymes de phase II inhibition par interaction entre le sulforaphane et les thiols des cystéines de AP1, un facteur de transcription impliqué dans les cancers cutanés.

Par ailleurs, le sulforaphane est accumulé dans les cellules sous sa forme conjuguée au glutathion. En effet, la majeure partie des formes intracellulaires des isothiocyanates sont des formes dithiocarbamates résultant de la réaction de l'isothiocyanate sur le glutahion. Ainsi, 95 % du produit accumulé dans les cellules l'est sous cette forme.

De plus, il a été démontré que, bien que la forme dithiocarbamate soit la forme de stockage du sulforaphane, au niveau cellulaire (accumulation rapide en concentrations élevées), les cellules ne sont pas capables d'absorber directement cette forme si on la leur présente déjà préformée en lieu et place de l'isothiocyanate correspondant. Dans ce cas, il y aurait, dans un premier temps, hydrolyse extracellulaire du dithiocarbamate afin de revenir à l'isothiocyanate libre, forme qui peut alors être intégrée par la cellule puis convertie pour stockage sous forme de dithiocarbamate.

L'accumulation rapide et à de fortes concentrations de ces isothiocyanates, stockés sous forme dithiocarbamate, semble jouer un rôle crucial dans leur capacité à induire le système des enzymes de phase II et un effet protecteur anti-cancer notamment.

Dans une forme de réalisation particulière de l'invention, ledit composé de formule générale (I) est du sulforaphane synthétique ou extrait.

Comme on l'a mentionné ci-avant, il est en effet avantageux de pouvoir bénéficier d'une quantité suffisante de sulforaphane stable et disponible afin de pouvoir effectuer les tests et mettre le produit sur le marché à des couts raisonnables. L'invention entend également proposer un procédé de fabrication de sulforaphane synthétique présentant un rendement et une pureté élevés. Le procédé de fabrication est décrit dans l'exemple de fabrication ci-dessous.

En particulier, selon l'invention, le composé de formule générale (I) est choisi dans le groupe constitué du sulforaphane, de l'oxomate, du sulforamate, des analogues du sulforamate, des thiourées issues de la réaction de couplage du sulforaphane avec une amine primaire ou secondaires et leurs formes oxydées, des composés issus de la réaction du sulforaphane avec un agent nucléophile comme un alcool ou un thiol, du sulforaphane oxydé au niveau de sa fonction sulfinyle ainsi que les dérivés issus du couplage avec un Nucléophile comme alcool ou thiol correspondants et les thiourées issues de la réaction du sulforaphane oxydé au niveau de sa fonction sulfinyle avec une amine primaire ou secondaire, du sulforamate oxydé au niveau de sa fonction sulfinyle, des alcools ou thiols issus de la réaction de l'oxomate et avec un agent nucléophile alcool ou thiol, des thiourées issues de la réaction de l'oxomate avec une amine primaire ou secondaire.

Par sa structure intéressante, le sulforamate pourrait également avoir un intérêt dans les applications susdites, mais il faudrait pouvoir en disposer à volonté. En effet, cette molécule présente une structure "simplifiée" mimant celle du métabolite naturel du sulforaphane formé in-vivo par réaction du sulforaphane sur le S d'une cystéine du glutathion.

Toutes ces molécules présentent une structure dérivée ou analogue à celle du sulforaphane ou de son métabolite. Dès lors, il est envisageable au sens de l'invention que tous ces analogues et dérivés du surlforaphane présentent un effet bénéfique sur le traitement et/ou la prévention de la lucite. En effet, il est possible d'envisager le couplage d'un large panel d'amines choisies comme molécules de référence sur le sulforaphane, l'oxomate pour former des dérivés. Ces amines seraient alors choisies pour leur effets de protection contre les UV et leur effets anti-inflammatoire, car elles sont supposées avoir un effet en ce sens qui pourrait venir renforcer l'effet.

En outre, certains métabolites du sulforaphane, tel que le produit de couplage avec le glutathion, mentionné ci-avant, ne peuvent pas être stockés directement par la cellule si ils lui sont présentés. Ils doivent d'abord être clivés in-vivo pour revenir au sulforaphane qui est phagocyté par les cellules, pour être ensuite stocké sous forme couplée au glutathion. Ainsi, de manière analogue, les structures sulforamate, nucléophile (amine, ...) couplé au sulforaphane ou à l'oxomate, selon la présente invention, chez l'homme, seraient également clivées lors de la métabolisation, ce qui provoquerait d'une part une libération de sulforaphane à effet de protection des cellules contre les UV, dommages à l'ADN etc... et d'autre part de l'amine libre qui pourrait aussi jouer son rôle protecteur. Dès lors, ces dérivés du sulforaphane permettent plusieurs possibilités d'action : soit la thiourée est active en elle-même, soit elle est métabolisée pour libérer 2 molécules ayant chacune un effet protecteur.

Par exemple, il est possible d'envisager le couplage d'amines connues pour avoir un effet filtre UV (par exemple : acide para-amino benzoïque ou anthranilate) ou encore le couplage avec des diamines comme la pipérazine pour obtenir une molécules portant deux fonctions thiourées avec 2 unités "sulforaphane" et une diamine centrale.

Dans une forme de réalisation avantageuse, dès lors, dans le procédé selon l'invention, l'amine est une amine primaire présentant la formule générale HNR₅R₆ dans laquelle R₅ représente un atome d'hydrogène et R₆ un groupement méthylsulfinylbutyle. Ceci permet d'atteindre par exemple la 1,3-bis-(4-(méthylsulfinyl)butyl)-thiourée.

Avantageusement, l'amine primaire présente la formule générale HNR₅R₆ dans laquelle R₅ représente un atome d'hydrogène et R₆ un groupement alkyle, alcényle, alcynyle, linéaire, cyclique ou ramifié comportant éventuellement un ou plusieurs hétéroatomes.

Dans une variante au sens de l'invention, ladite amine est une amine secondaire qui présente la formule générale HNR₅R₆ dans laquelle R₅ et R₆ représentent indépendamment l'un de l'autre un groupement alkyle, alcényle, alcynyle, linéaire, cyclique ou ramifié comportant éventuellement un ou plusieurs hétéroatomes.

Dans une forme de réalisation particulièrement avantageuse selon l'invention, ledit composé de formule générale (I) est incorporé dans une formulation galénique pour une application topique.

Plus particulièrement, lorsque ledit composé de formule générale (I) est du sulforaphane, il est avantageux qu'il soit incorporé dans une formulation galénique comprenant une matrice comprenant au moins un ester choisi dans le groupe constitué des acétates, des benzoates , des salicylates, de l'ester 610, des triglycerides capryliques/ capriques/succiniques, des stéarates et isostéarates, des éthylhexanoates, des palmitates, des isononanoates, des oleates, des néopentanoates, des myristates et analogues.

En effet, le sulforaphane est connu pour être une molécule très sensible à divers facteurs tels que la chaleur, les agents nucléophiles, et donc pour se dégrader rapidement en présence de ces facteurs, ce qui limite fortement son utilisation dans des préparations pour une application topique.

La matrice de choix qui permettra l'utilisation de sulforaphane dans une préparation est donc une matrice à base d'esters purs et anhydres, compatibles avec les applications cosmétiques et pharmaceutiques envisagées. Ces composés permettent en effet d'allier solubilité (> 6 % (m/m)) et stabilité et sont aisément disponibles dans le commerce. Ces compositions peuvent également contenir un excipient cosmétiquement et/ou pharmaceutiquement acceptable tel qu'un méthylpolysiloxane.

D'autres formes d'utilisation selon l'invention sont mentionnées dans les revendications annexes.

La présente invention se rapporte donc particulièrement à un procédé de synthèse de sulforaphane comprenant principalement les étapes successives de synthèse du 4-méthylthiobutyronitrile (A), synthèse de la 4-méthylthiobutylamine (B), synthèse de la 4-méthylsulfinylbutylamine (C) et synthèse du sulforaphane (4-méthylsulfinylbutyl isothiocyanate) (D). Ces étapes sont indiquées ci-dessous.

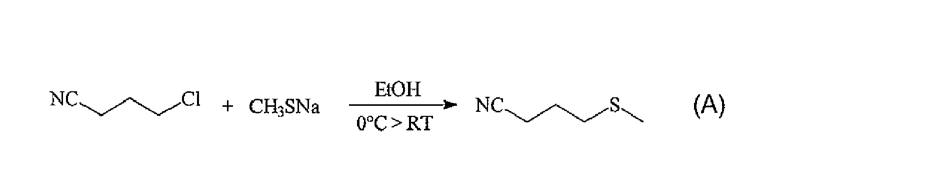

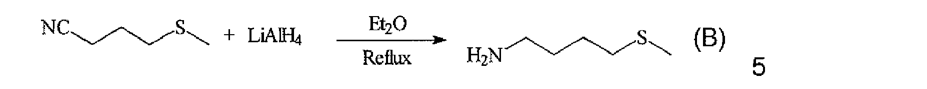

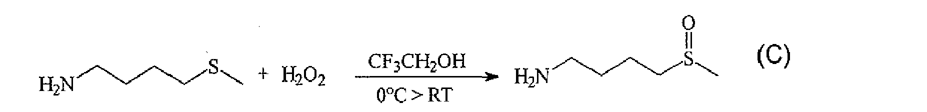

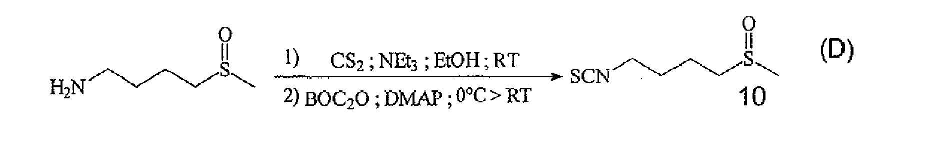

A partir de l'intermédiaire formé à l'étape (C), il est également possible de former un dérivé du sufloraphane, le sulforamate selon la réaction globale E.

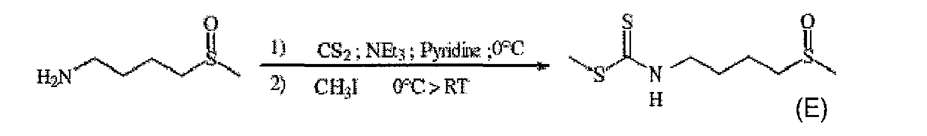

Egalement selon l'invention, une fois le sulforaphane formé, il peut alors réagir avec des amines pour former des dérivés du sulforaphane comme par exemple la 1,3-bis-(4-(méthylsulfinyl)butyl)-thiourée selon la réaction globale (F)

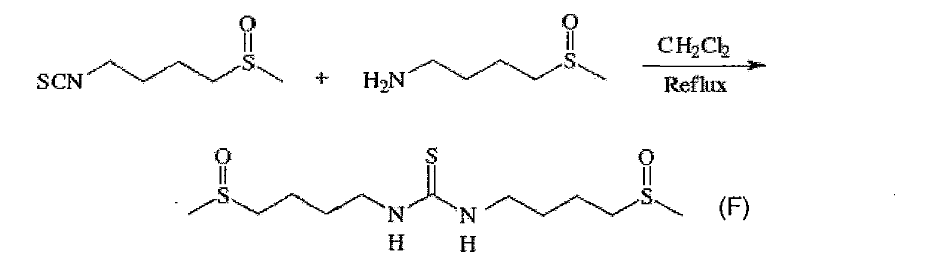

Un autre analogue du sufloraphane qui peut être produit selon l'invention est la *N*-(4-(méthylsulfinyl)butyl)pipéridine-1-carbothioamide par l'addition d'une amine, la pipéridine. Ce composé présente la formule A.

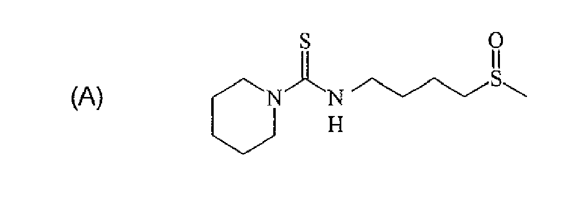

Un autre analogue du sufloraphane qui peut être produit selon l'invention est la *N*-(4-(méthylsulfinyl)butyl)morpholine-4-carbothioamide par l'addition d'une amine, la morpholine. Ce composé présente la formule B.

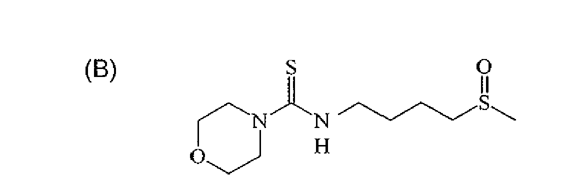

Encore un autre analogue du sufloraphane qui peut être produit selon l'invention est la 1-(1-benzylpiperidin-4-yl) -3-(4-(méthylsulfinyl)butyl)thiourée par l'addition d'une amine, la benzylpipéridine. Ce composé présente la formule (C).

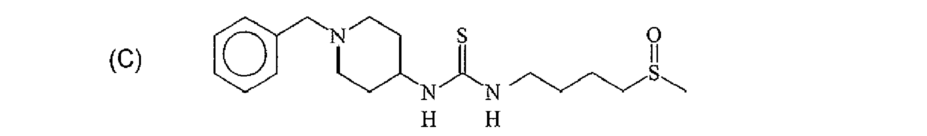

De plus, comme mentionné précédemment, l'invention prévoit également la fabrication de certains dérivés du sulforaphane comme par exemple la forme oxydée de celui-ci selon la réaction (G).

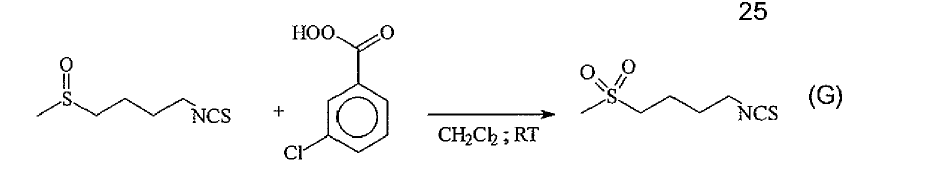

Au sens de l'invention, d'autres formes oxydées des dérivés du sulforaphane sont également obtenues selon l'invention d'une manière analogue à celle décrite ci-dessus. Ces composés oxydés comprennent le sulforamate oxydé ((4-méthylsulfonylbutylcarbamodithioate) présentant la formule suivante (D), les thiourée dérivant du couplage du sulforaphane oxydé sur une forme de l'amine intermédiaire de synthèse suivant les deux cas possibles 1-(4-(méthylsulfinyl)butyl)-3-(4-méthylsulfonyl)butyl) thiourée présentant la formule suivante (E) et 1,3-bis-(4-(methylsulfonyl)butyl)-thiourée présentant la formule suivante (F). En outre, d'autres thiourées peuvent être obtenues par un couplage d'amines primaires ou secondaires cycliques ou non sur le sulforaphane oxydé. Ces amines seront par exemple choisies en fonction des propriétés desdites amines, ces composés présentent alors la formule suivante (G). Si le couplage du sufloraphane oxydé est réalisé avec un alcool ou un thiol, on obtient par exemple dans le cas de l'éthanol, un composé selon la formule (H). Les formules (D) à (H) sont présentées au tableau.

**Tableau**

| N° formule | Produit |
|---|---|
| (D) | 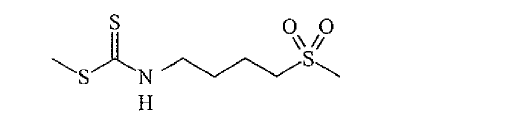 |
| (E) | 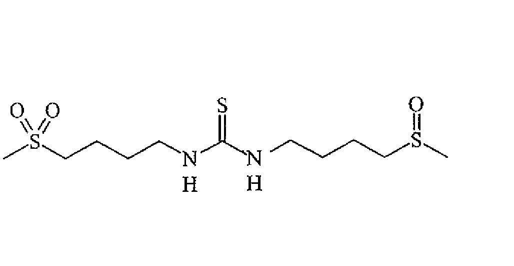 |
| (F) | 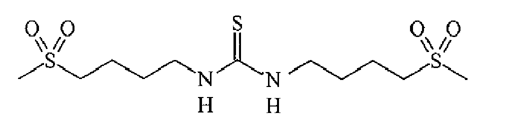 |
| (G) | 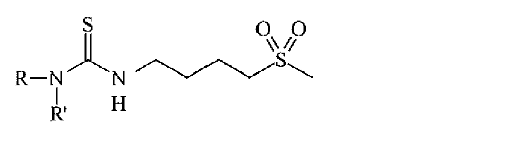 |
| (H) | 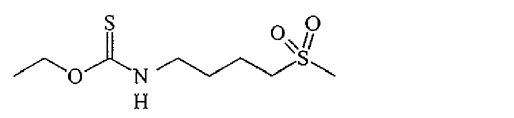 |

La présente invention procure donc également un procédé de synthèse d'oxomate selon la réaction (H).

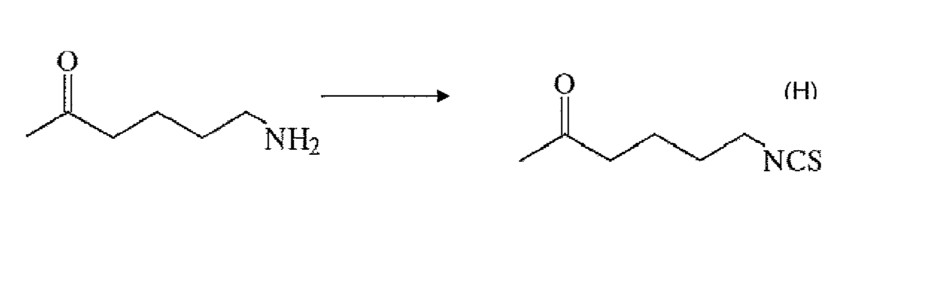

Divers analogues ou dérivés de l'oxomate qui peuvent être produits selon l'invention sont mentionnés ci-dessous. Par exemple, un des dérivés que l'on peut obtenir selon l'invention est la 1,3-bis(5-oxohexyl)thiourée, par dégradation de l'oxomate dans l'eau. Ce composé présente la formule générale (I).

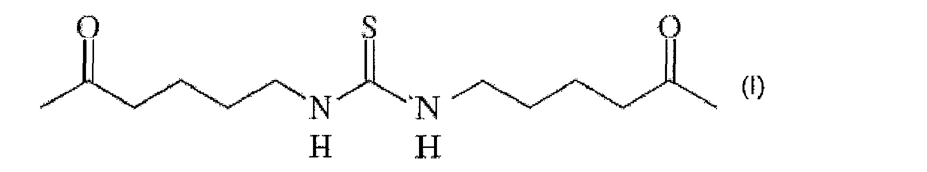

Par exemple un des dérivés que l'on peut obtenir selon l'invention est la 1-(4-(methylsulfinyl)butyl)-3-(5-oxohexyl)thiourée par l'addition d'une amine 4-methylsulfinylbutylamine. Ce composé présente la formule (J).

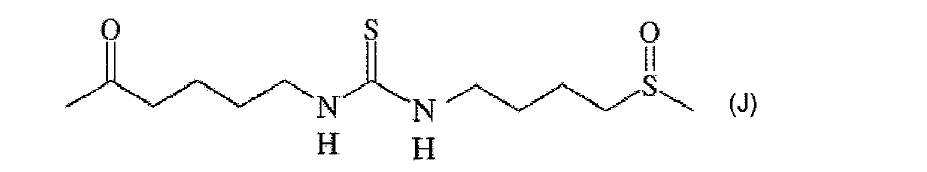

Par exemple un des dérivés que l'on peut obtenir selon l'invention est le 1-(4-(methylsulfonyl)butyl)-3-(5-oxohexyl)thiourée_par l'addition d'une amine 4-methylsulfonylbutylamine. Ce composé présente la formule (K).

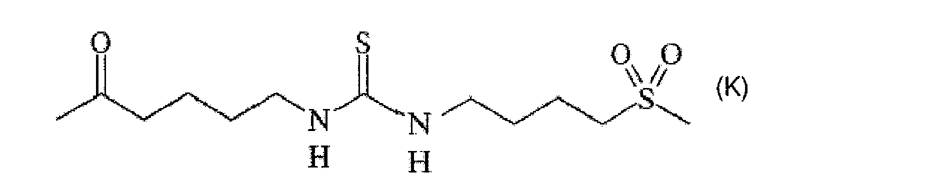

Par exemple un des dérivés que l'on peut obtenir selon l'invention par l'addition de morpholine *N*-(5-oxohexyl)morpholine-4-carbothioamide. Ce composé présente la formule (L). Par exemple avec

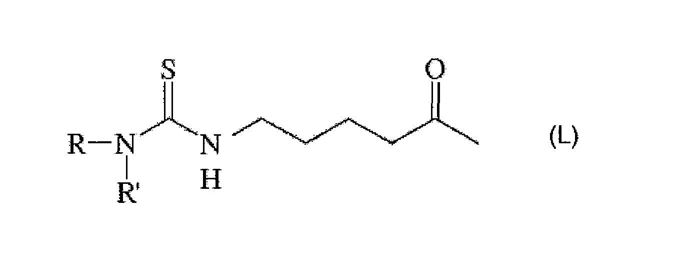

Par exemple, un des dérivés que l'on peut obtenir selon l'invention par l'addition d'un nucléophile comme un alcool ou un thiol, par exemple, l'éthanol est le *O*-ethyl-5-oxohexylcarbamothioate. Ce composé présente la formule (M).

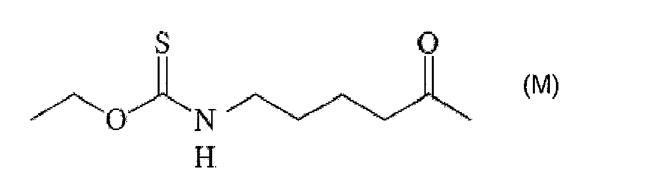

Il est bien entendu que la présente invention n'est en aucune façon limitée aux formes de réalisations décrites ci-dessus et que bien des modifications peuvent y être apportées sans sortir du cadre des revendications annexées.

## Revendications

1. Utilisation d'un composé de formule générale (1) pour la prévention et/ou le traitement de la lucite
R₇-R₁-R₄-R₂ (I)
dans laquelle R₄ est un groupement sulfinyle, sulfonyle, carbonyle, R₇ représente un groupement amino, isothiocyanato ou carbamothioate de formule -NH(C=S)-R₈ dans laquelle R₈ représente un groupe , amine primaire ou secondaire, alcoolate, ou thiolate et R₁ et R₂ représentent tous deux, indépendamment l'un de l'autre un groupe alkyle, aryle ou alkylaryle éventuellement substitué par un ou plusieurs hétéroatomes.

2. Utilisation selon la revendication 1 dans laquelle ledit composé de formule générale (I) est du sulforaphane synthétique ou extrait.

3. Utilisation selon la revendication 1 dans laquelle le composé de formule générale (I) est choisi dans le groupe constitué de l'oxomate, du sulforamate, des analogues du sulforamate, des thiourées issues de la réaction du sulforaphane avec une amine primaire ou secondaire et leurs formes oxydées, des produits issus de la réaction de nucléophiles tels les alcools ou thiols sur du sulforaphane du sulforaphane oxydé au niveau de sa fonction sulfinyle ainsi que des dérivés correspondants issus du couplage avec les alcools ou thiols et les thiourées issues de la réaction du sulforaphane oxydé au niveau de sa fonction sulfinyle avec une amine primaire ou secondaire, du sulforamate oxydé au niveau de sa fonction sulfinyle, des dérivés de couplage de Nu comme des alcools ou thiols sur l'oxomate, des thiourées issues de la réaction de l'oxomate avec une amine primaire ou secondaire.

4. Utilisation selon la revendication 3, dans laquelle ladite amine primaire présente la formule générale HNR₅R₆ dans laquelle R₅ représente un atome d'hydrogène et R₆ un groupement 4-méthylsulfinylbutyle.

5. Utilisation selon la revendication 3, dans laquelle l'amine primaire présente la formule générale HNR₅R₆ dans laquelle R₅ représente un atome d'hydrogène et R₆ un groupement alkyle, linéaire, cyclique ou ramifié comportant éventuellement un ou plusieurs hétéroatomes.

6. Utilisation selon la revendication 3, dans laquelle l'amine secondaire présente la formule générale HNR₅R₆ dans laquelle R₅ et R₆ représentent indépendamment l'un de l'autre un groupement alkyle, linéaire, cyclique ou ramifié comportant éventuellement un ou plusieurs hétéroatomes.

7. Utilisation selon la revendication 3, dans laquelle ledit agent nucléophile est un alcool ou un thiol.

8. Utilisation selon l'une quelconques des revendications 1 à 7 dans laquelle ledit composé de formule générale (I) est incorporé dans une formulation galénique pour une application topique.

9. Utilisation selon la revendication 8 dans laquelle ledit composé de formule générale (I) est du sulforaphane incorporé dans une formulation galénique comprenant une matrice comprenant au moins un ester choisi dans le groupe constitué des acétates, des benzoates , des salicylates, de l'ester 610, des triglycerides capryliques/ capriques/succiniques, des stéarates et isostéarates, des éthylhexanoates, des palmitates, des isononanoates, des oleates, des néopentanoates, des myristates et analogues.
